(19) Europäisches Patentamt
European Patent Office
Office européen des brevets

(11) Veröffentlichungsnummer: **0 102 586**
**B1**

(12) # EUROPÄISCHE PATENTSCHRIFT

(45) Veröffentlichungstag der Patentschrift: **11.12.85**

(51) Int. Cl.[4]: **C 07 D495/14**, G 03 C 1/68 // (C07D495/04, 335:00, 239:00)

(21) Anmeldenummer: **83108310.0**

(22) Anmeldetag: **24.08.83**

(54) **1,3-Diaza-9-thia-anthracen-2,4-dione und diese enthaltendes photopolymerisierbares Gemisch.**

(30) Priorität: **02.09.82 DE 3232621**

(43) Veröffentlichungstag der Anmeldung: **14.03.84 Patentblatt 84/11**

(45) Bekanntmachung des Hinweises auf die Patenterteilung: **11.12.85 Patentblatt 85/50**

(84) Benannte Vertragsstaaten: **AT BE CH DE FR GB IT LI NL SE**

(56) Entgegenhaltungen:
**EP-A- 0 005 750**
**CH-A- 599 569**
**JOURNAL OF HETEROCYCLIC CHEMISTRY, Band 18, November 1981, Seiten 1329-1334, Hetero Corporation, Tampa, US F. YONEDA et al.: "Synthesis of properties of 1-benzothiopyrano (2,3-d)-pyrimidine-2,4-(3H)diones (10-thia-5-deazaflavins)"**

(73) Patentinhaber: **HOECHST AKTIENGESELLSCHAFT**
**Postfach 80 03 20**
**D-6230 Frankfurt am Main 80 (DE)**

(72) Erfinder: **Wingen, Rainer, Dr.**
**Rauenthaler Weg 30**
**D-6000 Frankfurt 71 (DE)**
Erfinder: **Horn, Klaus, Dr.**
**Lessingstrasse 53**
**D-6238 Hofheim/Ts. (DE)**
Erfinder: **Lutz, Walter, Dr.**
**Eleonorenstrasse 130**
**D-6503 Mainz-Kastel (DE)**

**EP 0 102 586 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 1,3-Diaza-9-thia-anthracen-2,4-dione sowie ein photopolymerisierbares Gemisch, das als wesentliche Bestandteile (a) ein polymeres Bindemittel, (b) eine polymerisierbare Verbindung mit mindestens zwei endständigen ethylenisch ungesättigten Gruppen und einem Siedepunkt oberhalb 100 °C und (c) als Photoinitiator ein 1,3-Diaza-9-thia-anthracen-2,4-dion enthält.

Photopolymerisierbare Gemische aus den Bestandteilen (a) und (b) und einer mehrkernigen heterocyclischen Verbindung als Photoinitiator sind bekannt.

In der DE-C 20 27 467 (= GB-A 1 354 541) werden als Initiatoren bestimmte Derivate des Acridins und Phenazins beschrieben.

Aus der DE-C 20 39 861 (= US-A 3 765 898) sind ähnliche Gemische bekannt, die als Initiatoren Chinoxalin- oder Chinazolinderivate enthalten.

Alle diese Verbindungen zeigen hervorragende Initiatorwirkung unter Bestrahlung mit aktinischem Licht, insbesondere von Lichtquellen im nahen Ultraviolettbereich. Da in neuerer Zeit für Kopierzwecke wegen ihrer hohen Lichtausbeute verstärkt metallhalogeniddotierte Gasentladungslampen verwendet werden und diese gegenüber früher gebräuchlichen Lichtquellen, z. B. Quecksilberdampflampen, vermehrt Emissionen im sichtbaren Licht oberhalb 400 nm aufweisen, sind die Absorptionen der bekannten hochwirksamen Initiatoren nicht mehr optimal auf die Emissionen dieser Lichtquellen abgestimmt. Auch ist die Variationsmöglichkeit durch Substitution der bekannten heterocyclischen Initiatoren begrenzt, d. h. es ist nach den gängigen Herstellungsverfahren nur in begrenztem Maße möglich, andere Eigenschaften, wie Löslichkeit in wäßrigen oder organischen Lösungsmitteln oder Verträglichkeit mit unterschiedlichen photopolymerisierbaren Gemischen, nach Wunsch durch gezielte Synthese zu verändern.

In Journ. of Heterocyclic Chemistry 18, S. 1329 (1981) werden 1,3-Diaza-9-thia-anthracen-2,4-dione beschrieben, die in 6-Stellung unsubstituiert oder durch ein Chloratom oder eine Methylgruppe substituiert sind. Die Verbindungen werden dort auf ihr Oxydationsvermögen gegenüber Alkoholen untersucht. Andere Anwendungen werden dort nicht genannt.

Aufgabe der Erfindung war es, neue Photoinitiatoren mit ähnlich hoher Wirksamkeit wie die bekannten vorzuschlagen, deren Lichtabsorption weiter in den kurzwelligen sichtbaren Bereich hineinreicht und in die sich im Laufe ihres Synthesewegs gezielt bestimmte Substituenten einführen lassen, die den Endprodukten unterschiedliche gewünschte Eigenschaften, wie Löslichkeit und Verträglichkeit mit anderen Komponenten, verleihen.

Gegenstand der Erfindung ist ein photopolymerisierbares Gemisch, das als wesentliche Bestandteile

a) ein polymeres Bindemittel,

b) eine polymerisierbare Verbindung mit mindestens zwei endständigen ethylenisch ungesättigten Gruppen und mit einem Siedepunkt oberhalb 100 °C und

c) als Photoinitiator eine mehrkernige N-heterocyclische Verbindung

enthält.

Das erfindungsgemäße Gemisch ist dadurch gekennzeichnet, daß die N-heterocyclische Verbindung der Formel II

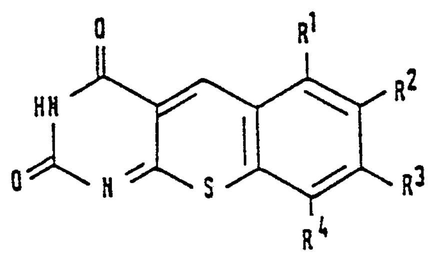

. (II)

entspricht, worin $R^1$, $R^2$, $R^3$ und $R^4$ gleich oder verschieden sind und einzeln jeweils ein Wasserstoff- oder Halogenatom, eine Carboxylgruppe, eine Alkyl-, Alkoxy- oder Alkoxycarbonylgruppe, worin die Alkylgruppen jeweils 1 bis 6 Kohlenstoffatome enthalten, oder jeweils zwei der Reste $R^1$ bis $R^4$ zusammen einen ankondensierten aromatische Rest bedeuten.

Erfindungsgemäß werden ferner neue 1,3-Diaza-9-thiaanthracen-2,4-dione der allgemeinen Formel II

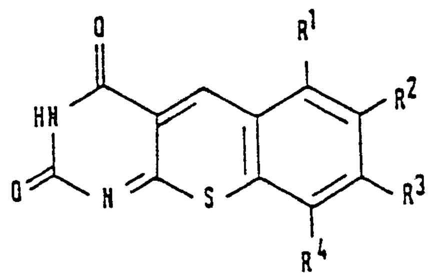

(II)

vorgeschlagen, worin

$R^1$, $R^3$ und $R^4$ gleich oder verschieden sind und einzeln jeweils ein Wasserstoff- oder Halogenatom, eine Carboxylgruppe, eine Alkyl-, Alkoxy-, oder Alkoxycarbonylgruppe, worin die Alkylgruppen jeweils 1 bis 6 Kohlenstoff atome enthalten, bedeuten und

$R^2$ eine Carboxyl-, eine Alkoxy- oder Alkoxycarbonylgruppe mit jeweils 1 bis 6 Kohlenstoffatomen in der Alkoxygruppe ist

oder jeweils zwei der Reste

$R^1$, $R^2$, $R^3$ und $R^4$ gemeinsam einen ankondensierten aromatischen Rest

bedeuten.

Die neuen Verbindungen können auf dem in der anliegenden Formelzeichnung skizzierten Weg hergestellt werden.

Sie absorbieren Licht im Spektralbereich von etwa 425 bis 480 nm und wirken bei Bestrahlung in diesem Spektralbereich als aktive Radikalstarter für die Photopolymerisation von Vinylverbindungen, auch in Gegenwart von Sauerstoff. Die neuen Photoinitiatoren haben die Eigenschaft, die thermische Polymerisation solcher Verbindungen in Abwesenheit von aktinischer Strahlung nicht zu initiieren. Sie sind somit ideal zur Herstellung lagerfähiger Kopierschichten geeignet.

Es ist hierbei von Vorteil, daß bei der Herstellung der Verbindungen (II) in überschaubaren Reaktionen gut zugängliche literaturbekannte Edukte verwendet werden, breite Variation der Substituenten $R^1$-$R^4$ möglich ist und Reinigungsoperationen erst auf der Endstufe erforderlich sind.

Die Alkyl- und Alkoxygruppen, die als Substituenten in Betracht kommen, haben vorzugsweise 1 bis 3 Kohlenstoffatome ; Methyl- und Methoxygruppen werden besonders bevorzugt.

Als Halogenatome werden Fluor-, Chlor- und Bromatome, insbesondere Chloratome, bevorzugt. In den Alkoxycarbonylgruppen können die gleichen Alkoxygruppen vorliegen wie oben angegeben. Als ankondensierte aromatische Reste werden Benzoreste bei weitem bevorzugt.

Von den Verbindungen der Formel II werden diejenigen besonders bevorzugt, in denen mindestens einer der Reste $R^1$ bis $R^4$ ein Wasserstoffatom ist. Besonders bevorzugt werden Verbindungen, in denen zwei oder drei dieser Reste, insbesondere der Reste $R^1$ bis $R^3$ Wasserstoffatome sind. Wenn zwei der Reste eine Benzogruppe bedeuten, sind die beiden anderen bevorzugt Wasserstoffatome.

Die Photoinitiatoren werden im allgemeinen in einer Menge von 0,01 bis 10, bevorzugt von 0,1 bis 5 Gew.-%, bezogen auf die Bestandteile der photopolymerisierbaren Schicht, zugesetzt.

Für die Zwecke der Erfindung geeignete photopolymerisierbare Monomere sind bekannt und z. B. in den USA-Patentschriften 2 760 863 und 3 060 023 beschrieben.

Bevorzugte Beispiele sind Acryl- und Methacrylsäureester, wie Diglycerindiacrylat, Polyethylenglykoldimethacrylat, Acrylate und Methacrylate von Trimethylolethan, Trimethylolpropan und Pentaerythrit und von mehrwertigen alicyclischen Alkoholen. Mit Vorteil werden auch Umsetzungsprodukte von Diisocyanaten mit Partialestern mehrwertiger Alkohole eingesetzt. Derartige Monomere sind in den DE-A 20 64 079, 23 61 041 und 28 22 190 beschrieben.

Der Mengenanteil der Schicht an Monomeren beträgt im allgemeinen etwa 10 bis 80, vorzugsweise 20 bis 60 Gew.-%.

Als Bindemittel können eine Vielzahl löslicher organischer Polymerisate Einsatz finden. Als Beispiele seien genannt : Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.

Ferner kommen als Bindemittel Naturstoffe oder umgewandelte Naturstoffe in Betracht, z. B. Gelatine und Celluloseether.

Mit besonderem Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrigalkalischen Entwickern lassen. Derartige Bindemittel können z. B. die folgenden Gruppen enthalten : $-COOH$, $-PO_3H_2$, $-SO_3H$ ; $-SO_2NH-$ und $-SO_2-NH-CO-$.

Als Beispiele hierfür seien genannt : Maleinatharze, Polymerisate aus N-(p-Tolyl-sulfonyl)-carbaminsäure-(β-methacryloyloxy-ethyl)ester und Mischpolymerisate dieser und ähnlicher Monomer mit anderen Monomeren sowie Styrol-Maleinsäureanhydrid-Mischpolymerisate. Alkylmethacrylat-Methacrylsäure-Mischpolymerisate und Mischpolymerisate aus Methacrylsäure, Alkylmethacrylaten und Methylmethacrylat und/oder Styrol, Acrylnitril u. a., wie sie in den DE-A 20 64 080 und 23 63 806 beschrieben sind, werden bevorzugt.

Die Menge des Bindemittels beträgt im allgemeinen 20 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind :

Inhibitoren zur Verhinderung der thermischen Polymerisation der Monomeren, Wasserstoffdonatoren, die sensitometrischen Eigenschaften derartiger Schichten modifizierende Stoffe, Farbstoffe, gefärbte und ungefärbte Pigmente, Farbbildner, Indikatoren, Weichmacher usw.

Diese Bestandteile sind zweckmäßig so auszuwählen, daß sie in dem für den Initierungsvorgang wichtigen aktinischen Strahlungsbereich möglichst wenig absorbieren.

Als aktinische Strahlung soll im Rahmen dieser Beschreibung jede Strahlung verstanden werden, deren Energie mindestens der des kurzwelligen sichtbaren Lichts entspricht. Geeignet ist langwellige UV-Strahlung, aber auch Elektronen-, Röntgen- und Laserstrahlung.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Kopiermaterial auf dem Reproduktionsgebiet.

Die detaillierte Beschreibung der Erfindung beschränkt sich auf dieses Anwendungsgebiet, jedoch ist die Erfindung nicht hierauf beschränkt. Als Anwendungsmöglichkeiten auf diesem Gebiet seien genannt : Kopierschichten für die photomechanische Herstellung von Druckformen für den Hochdruck, den Flachdruck, den Tiefdruck, den Siebdruck, von Reliefkopien, z. B. Herstellung von Texten in Blindenschrift, von Einzelkopien, Gerbbildern, Pigmentbildern usw. Weiter sind die Gemische zur photomechanischen Herstellung von Ätzreservagen, z. B. für die Fertigung von Namensschildern, von kopierten Schaltungen und für das Formteilätzen, anwendbar. Besondere Bedeutung haben die erfindungsgemäßen Gemische als Kopierschichten für die photomechanische Herstellung von Flachdruckformen und von Ätzreservagen, insbesondere als vorsensibilisierte Materialien.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Forteilätzen, für die Herstellung kopierter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen, Ebenso ist es für die Herstellung von Trockenresist geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z. B. Wachse, Polyvinylalkohol, Polyphosphate, Zucker usw.

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Die Herstellung der lichtempfindlichen Materialien unter Verwendung des erfindungsgemäßen Gemischs erfolgt in bekannter Weise.

So kann man dieses in einem Lösungsmittel aufnehmen und die Lösung bzw. Dispersion durch Gießen, Sprühen, Tauchen, Antrag mit Walzen usw. auf den vorgesehenen Träger als Film antragen und anschließend antrocknen. Dicke Schichten (z. B. von 250 μm und darüber) werden vorteilhaft durch Extrudieren oder Verpressen als selbsttragende Folie hergestellt, welche dann ggf. auf den Träger laminiert wird. Im Falle von Trockenresist werden Lösungen des Gemischs auf transparente Träger aufgebracht und angetrocknet. Die lichtempfindlichen Schichten — Dicke etwa zwischen 10 und 100 μm — werden dann gleichfalls zunächst durch Laminieren zusammen mit dem temporären Träger auf das gewünschte Substrat auflaminiert.

Die Verarbeitung der Kopiermaterialien wird in bekannter Weise vorgenommen. Zur Entwicklung werden sie mit einer geeigneten Entwicklerlösung, bevorzugt einer schwach alkalischen wäßrigen Lösung, behandelt, wobei die unbelichteten Anteile der Schicht entfernt werden und die belichteten Bereiche der Kopierschicht auf dem Träger zurückbleiben.

Im folgenden werden Beispiele für das erfindungsgemäße Gemisch angegeben. Dabei wird zunächst die Herstellung einer Anzahl neuer Photoinitiatoren gemäß der Erfindung beschrieben.

In den Herstellungsvorschriften und den nachfolgenden Beispielen stehen Gewichtsteile (Gt) und Volumenteile (Vt) im Verhältnis von g zu ccm. Sofern nicht anders angegeben, verstehen sich Prozent- und Mengenverhältnisse in Gewichtseinheiten.

Der überwiegende Teil der in dem erfindungsgemäßen Gemisch enthaltenen Initiatoren ist aus der Literatur nicht bekannt. Ihre Darstellung erfolgt nach folgender allgemeiner Arbeitsvorschrift :

A) Allgemeine Vorschrift zur Herstellung der Verbindungen I :

1 mol 2,4,6-Trichlorpyrimidin-5-carbaldehyd wird in Tetrahydrofuran bei – 50 bis – 30 °C mit 1 mol Arylthiol sowie 1,1 Mol Triethylamin umgesetzt, das Aminhydrochlorid durch Filtration abgetrennt und der nach Abdestillieren des Lösungsmittels verbleibende Rückstand durch Verrühren mit einem geeigneten Lösungsmittel zur Kristallisation gebracht. Die erhaltenen 2,4-Di-chlor-6-arylthio-pyrimidin-5-carbaldehyde (Verbindungen 1 bis 31) zersetzen sich im Festzustand und werden direkt weiter umgesetzt ; in Tabelle 1 sind daher Rohausbeuten angegeben.

Tabelle 1

Verbindungen der Formel I

| Nr. | $R^1$ | $R^2$ | $R^3$ | $R^4$ | Ausb. % |
|---|---|---|---|---|---|
| 1 | H | H | H | H | 71,9 |
| 2 | H | H | H | $CH_3$ | 34,8 |
| 3 | H | H | $CH_3$ | H | 52,5 |
| 4 | H | $CH_3$ | H | H | 63,8 |
| 5 | H | F | H | H | 56,1 |
| 6 | H | H | H | Cl | 54,1 |
| 7 | H | H | Cl | H | 56,1 |
| 8 | H | Cl | H | H | 40,7 |
| 9 | H | Br | H | H | 69,1 |
| 10 | H | H | H | $OCH_3$ | 66,0 |
| 11 | H | H | H | COOH | 75,9 |
| 12 | H | H | H | $COOCH_3$ | 61,0 |
| 13 | H | COOH | H | H | 31,3 |
| 14 | H | H | $CH_3$ | $CH_3$ | 25,5 |
| 15 | H | $CH_3$ | H | $CH_3$ | 57,5 |
| 16 | $CH_3$ | H | H | $CH_3$ | 56,8 |
| 17 | H | H | Cl | $CH_3$ | 72,2 |
| 18 | H | Cl | H | $CH_3$ | 65,9 |
| 19 | Cl | H | H | $CH_3$ | 67,7 |
| 20 | H | H | $CH_3$ | Cl | 82,1 |
| 21 | H | $CH_3$ | H | Cl | 66,9 |
| 22 | H | H | Cl | Cl | 39,1 |
| 23 | Cl | H | H | Cl | 67,6 |
| 24 | Cl | H | Cl | H | 71,7 |
| 25 | $CH_3$ | $CH_3$ | H | $CH_3$ | 45,2 |
| 26 | $CH_3$ | Cl | H | $CH_3$ | 57,5 |
| 27 | $CH_3$ | Cl | H | Cl | 56,8 |
| 28 | H | Cl | Cl | Cl | 53,7 |
| 29 | Cl | Cl | H | Cl | 33,5 |
| 30 | H | H | - Benzo - | | 40,1 |
| 31 | - Benzo - | | H | H | 76,1 |

Allgemeine Vorschrift zur Herstellung der Verbindungen der Formel II:

1 Gt einer der Verbindungen 1 bis 31 wird bei 70 °C in 20 Gt $H_2SO_4$ (98 %) eingestreut, bis zum Ende der HCl-Entwicklung auf 110 bis 150 °C erhitzt, auf 120 Gt Eis gegossen, mit Alkali bei Temperaturen von 30 bis 40 °C auf pH 4 gebracht, der Feststoff abgetrennt, getrocknet und aus Dimethylformamid umkristallisiert.

(Siehe Tabelle 2 Seite 6 f.)

Tabelle 2

1,3-Diaza-9-thia-anthracen-2,4-dione der Formel II

| Nr. | $R^1$ | $R^2$ | $R^3$ | $R^4$ | Schmp. °C | Ausb. % |
|---|---|---|---|---|---|---|
| 32 | H | H | H | H | 290 | 62,3 |
| 33 | H | H | $CH_3$ | H | 300 | 68,4 |
| 34 | H | H | H | $CH_3$ | 300 | 64,8 |
| 35 | H | F | H | H | 280 | 11,5 |
| 36 | H | Cl | H | H | 300 | 17,6 |
| 37 | H | H | H | Cl | 290 | 50,7 |
| 38 | H | Br | H | H | 280 | 10,5 |
| 39 | H | H | H | COOH | 280 | 21,6 |
| 40 | H | H | H | $COOCH_3$ | 270 | 5,2 |
| 41 | $CH_3$ | H | H | $CH_3$ | 270 | 22,2 |
| 42 | H | $CH_3$ | H | $CH_3$ | 310 | 6,6 |
| 43 | H | H | $CH_3$ | $CH_3$ | 270 | 6,8 |
| 44 | Cl | H | H | $CH_3$ | 290 | 52,7 |
| 45 | H | Cl | H | $CH_3$ | 300 | 35,9 |
| 46 | H | $CH_3$ | H | Cl | 290 | 47,8 |
| 47 | Cl | H | Cl | H | 210 | 42,3 |
| 48 | Cl | H | H | Cl | 270 | 49,7 |
| 49 | $CH_3$ | $CH_3$ | H | $CH_3$ | 300 | 9,7 |
| 50 | $CH_3$ | Cl | H | $CH_3$ | 350 | 54,[illegible] |
| 51 | $CH_3$ | Cl | H | Cl | 280 | 11,2 |
| 52 | Cl | Cl | H | Cl | 280 | 3,2 |
| 53 | - Benzo - | | H | H | 300 | 42,8 |
| 54 | H | H | - Benzo - | | 290 | 42,8 |

Die Starteraktivität der Verbindungen Nr. 32 bis 54 ist in einer Reihe von Beispielen tabellarisch zusammengefaßt.

## Beispiel 1

Eine Lösung von
4,0 Gt eines Methylmethacrylat/Methacrylsäure-Copolymerisats (Säurezahl ca. 110),
4,0 Gt Trimethylolethantriacrylat,
0,08 Gt eines blauen Azofarbstoffs, erhalten durch Kuppeln von 2,4-Dinitro-6-chlorbenzoldiazoniumsalz mit 2-Methoxy-5-acetylamino-N-cyanoethyl-N-hydroxyethyl-anilin, und
0,21 Gt Initiator in
38 Gt Ethylenglykolmonoethylether und
18 Gt Butylacetat
wird auf elektrolytisch aufgerauhtes und anodisch oxydiertes 0,3 mm starkes Aluminium so aufgeschleudert und getrocknet, daß ein Schichtgewicht von 25 g/m² erhalten wird.

Nach dem Trocknen wird die Photopolymerschicht mit einer Lösung von
5 Gt Polyvinylalkohol (12 % Restacetylgruppen ; K-Wert 8) in
95 Gt entsalztem Wasser
überschichtet und getrocknet, so daß eine abziehbare Deckschicht von ca. 5 g/m² entsteht.

Anschließend wird mit einer 5 kW-Metallhalogenidlampe 40 Sekunden unter einem 13-stufigen Belichtungskeil belichtet. Nach dem Belichten wird die Platte 1 Minute auf 120 °C erwärmt.

Die belichtete Photopolymerschicht wird ca. 1 Minute mittels eines Plüschtampons mit einem Entwickler aus

1,5 Gt Natriummetasilikat × 9 $H_2O$,
0,01 Gt Polyoxyethylenether von Kokosfettalkohol mit etwa 8 Oxyethyleneinheiten und
98,3 Gt entsalztem Wasser

entwickelt.

Nach Wasserspülung wird mit 1 %iger Phosphorsäure sauer gestellt und mit Fettfarbe eingefärbt.

Nach dem Einfärben wird mit handelsüblicher Konservierungslösung gummiert und getrocknet. Auf einer Offsetdruckmaschine werden Auflagen um 100 000 erhalten.

Es werden folgende Lichtempfindlichkeiten erhalten :

| Verbindung | entwickelte Vollstufen |
|---|---|
| 32 | 2 |
| 33 | 3 |
| 34 | 2 |
| 35 | 1 |
| 36 | 1 |
| 37 | 5 |
| 38 | 2 |
| 39 | 3 |
| 40 | 2 |
| 41 | 2 |
| 42 | 1 |
| 43 | 2-3 |
| 44 | 4 |
| 45 | 4 |
| 46 | 4 |
| 47 | 2-3 |
| 48 | 5 |
| 49 | 2 |
| 50 | 2 |
| 51 | 1 |
| 52 | 2 |
| 53 | 1 |
| 54 | 2-3 |

## Beispiel 2

7 Lösungen von jeweils

5,6 Gt des Umsetzungsproduktes aus 1 mol 2,2,4-Trimethyl-hexamethylendiisocyanat und 2 mol 2-Hydroxy-ethylmethacrylat,
6,5 Gt eines Terpolymerisats aus Styrol, n-Hexylmethacrylat und Methacrylsäure (10 : 60 : 30),
0,2 Gt einer der Verbindungen 33, 37, 39, 44, 45, 46 und 48,
0,15 Gt Triethylenglykol-dimethacrylat und
0,035 Gt des in Beispiel 1 angegebenen blauen Azofarbstoffs in
30 Gt Butanon und
0,5 Gt Ethylalkohol

werden nacheinander auf eine 25 µm starke Polyethylenterephthalatfolie so aufgeschleudert, daß eine 25 µm (30 g/$m^2$) dicke Schicht erhalten wird. Anschließend wird 2 Minuten bei 100 °C im Trockenschrank nachgetrocknet.

Um die erhaltenen Schichten vor Verschmutzung durch Staub und Beschädigungen zu schützen, werden sie mit einer 20-25 µm starken Deckfolie, die an der Schicht weniger stark haftet als die Polyester-Trägerfolie, abgedeckt. Sie können so über einen längeren Zeitraum gelagert werden.

Die Kupferoberfläche einer mit 35 µm starker Kupferfolie kaschierten Phenoplast-Schichtstoffplatte wird mechanisch mit Bimsmehl oder mit einer Bürstenmaschine gereinigt und nach intensivem Abspülen mit Wasser mit ölfreier Luft trockengeblasen.

Auf die vorgereinigten Cu-Platten wird der Trockenresist — nach Abziehen der Deckfolie — mit Hilfe eines mit beheizten Walzen bestückten Laminators bei 120 °C und mit einer Geschwindigkeit von 1,5 m/min laminiert.

Alle sieben Proben werden anschließend durch die Trägerfolie unter einem 13-stufigen Belichtungskeil mit Dichteinkrementen von 0,15 mit einer 5 kW-MH-Lampe 10, 20 und 40 Sekunden belichtet.

Die Keilstufe 0 entspricht der optischen Dichte 0,05 (Eigen-Absorption des Filmmaterials).

Nach Abziehen der Trägerfolie werden die Platten einer Sprühentwicklung mit 0,8 %iger Sodalösung unterworfen. Die Entwicklungszeit beträgt ca. 60 Sekunden bei 23 °C.

Um die Entwicklerresistenz zu prüfen — ein Test, um festzustellen, ob die Keilstufen voll vernetzt sind — wurden die Proben nach einer Belichtung von 20 Sekunden der 3-fachen Entwicklungszeit — also 180 Sekunden — unterworfen.

Die voll vernetzten Keilstufen der Trockenresistschichten, die sich nur durch den Photoinitiator unterscheiden, sind in folgender Tabelle zusammengefaßt:

| Verbindung | Vollstufen im Sprühgerät bei | | | Vollstufen bei 180 Sekunden Entwicklung |
|---|---|---|---|---|
| | 10 Sek. | 20 Sek. | 40 Sek. | |
| | Belichtung | | | |
| 33 | - | 1 | 3 | - |
| 37 | 2 | 4 | 6 | 3 |
| 39 | - | 1 | 3 | - |
| 44 | 1 | 3 | 5 | 2 |
| 45 | 1 | 3 | 5 | 2 |
| 46 | 1 | 3 | 5 | 2 |
| 48 | 2 | 4 | 6 | 3 |

Beispiel 3

Je 0,4 g der Verbindungen 37 und 48 werden wie in Beispiel 2 beschrieben in Photopolymergemische eingearbeitet und die Lösungen auf 25 µm starke Polyethylenterephthalatfolien so aufgeschleudert, daß 25 µm (30 g/m$^2$) dicke Schichten erhalten werden.

Die Schichten werden in gleicher Weise wie in Beispiel 2 auf die gereinigte Cu-Oberfläche von 10 × 15 cm großen Cu-Phenolharz-Verbundplatten aufgebracht, durch die Trägerfolie belichtet und mit 0,8 %iger wäßriger Sodalösung entwickelt.

Es ergeben sich folgende vollvernetzte Keilstufen (zum Vergleich dahinter: Vollstufen bei Zugabe von je 0,6 g/Lösung):

| Verbindung | Vollstufen bei 0,4 Initiator Belichtungszeit, Sekunden | | | Vergleich: bei 0,6 g Initiator Belichtungszeit, Sekunden | | |
|---|---|---|---|---|---|---|
| | 10 | 20 | 40 | 10 | 20 | 40 |
| 37 | 3 | 6 | 8 | 2 | 3 | 5 |
| 48 | 3 | 5 | 7 | 2 | 4 | 6 |

Beispiel 4

Eine Lösung von

1,0 Gt Trimethylolethantriacrylat,

1,4 Gt eines Terpolymerisats aus n-Hexylmethacrylat/Methylmethacrylat/Methacrylsäure (50 : 25 : 25) mit einer Säurezahl von ca. 160,

0,02 Gt Sudanblau II und

0,05 Gt Verbindung 37 in

6,0 Gt Butanon

wird auf eine gesäuberte Einstufen-Zinkätzplatte so aufgeschleudert und getrocknet, daß ein Schichtgewicht von ca. 10 g/m$^2$ erhalten wird.

Anschließend wird das Kopiermaterial mit einem Überzug von 1-2 µm Polyvinylalkohol versehen, getrocknet und 40 Sekunden unter einer Positiv-Vorlage mit einer MH-Lampe von 5 kW belichtet. Die Zinkplatte wird 45 Sekunden mit einem Entwickler aus

1,5 Gt Natriummetasilikat-Nonahydrat,
0,3 Gt Polyglykol 6000,
0,3 GT Lävulinsaüre und
97,6 Gt entsalztem Wasser
entwickelt.

Nach gründlicher Wasserspülung wird 5 Minuten mit 10 %iger Salpetersäure unter Zusatz von Flankenschutzmittel geätzt. Die gehärtete Photopolymerschicht wird mit Ethylenglykolmonobutylether entfernt. Die so entstandene Druckform eignet sich für den Qualitätsbuchdruck.

Beispiel 5

Eine Beschichtungslösung wie in Beispiel 4, jedoch mit 0,05 Gt der Verbindung 48 anstelle der Verbindung 37 wird auf eine 25 µm starke Polyethylenterephthalatfolie durch Gießen so aufgetragen, daß eine 20 µm (26 g/m²) dicke Schicht erhalten wird. Anschließend wird 2 Minuten bei 100 °C im Trockenschrank nachgetrocknet.

Die getrocknete Schicht wird zusammen mit der Polyesterfolie mittels eines Laminators bei höchstem Andruck und einer Temperatur von 115 °C mit einer Geschwindigkeit von 1 m/min auf Siebdruckgewebe VS-Monoprint P 77 der Firma Verseidag, Krefeld, auflaminiert.

Anschließend wird durch die Polyesterfolie 60 Sekunden mit einer 5 kW-MH-Lampe unter einer Positiv-Vorlage belichtet.

Nach Entfernen der Polyesterfolie werden mit den in Beispiel 4 beschriebenen Entwickler innerhalb von 45 Sekunden im Schaukelbad die unvernetzten Bildbereiche entfernt. Nach gründlicher Wasserspülung und Trocknung ist die Siebdruckform gebrauchsfertig.

**Patentansprüche**

1. Photopolymerisierbares Gemisch, das als wesentliche Bestandteile
a) ein polymeres Bindemittel,
b) eine polymerisierbare Verbindung mit mindestens zwei endständigen ethylenisch ungesättigten Gruppen und mit einem Siedepunkt oberhalb 100 °C und
c) als Photoinitiator eine mehrkernige N-heterocyclische Verbindung
enthält, dadurch gekennzeichnet, daß die N-heterocyclische Verbindung der Formel II

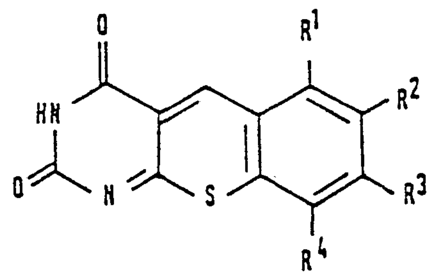

(II)

entspricht, worin $R^1$, $R^2$, $R^3$ und $R^4$ gleich oder verschieden sind und einzeln jeweils ein Wasserstoff- oder Halogenatom, eine Carboxylgruppe, eine Alkyl-, Alkoxy- oder Alkoxycarbonylgruppe, worin die Alkylgruppen jeweils 1 bis 6 Kohlenstoffatome enthalten, oder jeweils zwei der Reste $R^1$ bis $R^4$ zusammen einen ankondensierten aromatischen Rest bedeuten.

2. Photopolymerisierbares Gemisch nach Anspruch 1, dadurch gekennzeichnet, daß es eine Verbindung der Formel II enthält, worin einer der Reste $R^1$ bis $R^4$ ein Wasserstoffatom ist.

3. Photopolymerisierbares Gemisch nach Anspruch 2, dadurch gekennzeichnet, daß es eine Verbindung der Formel II enthält, worin zwei oder drei der Reste $R^1$ bis $R^3$ Wasserstoffatome sind.

4. 1,3-Diaza-9-thia-anthracen-2,4-dione der allgemeinen Formel II

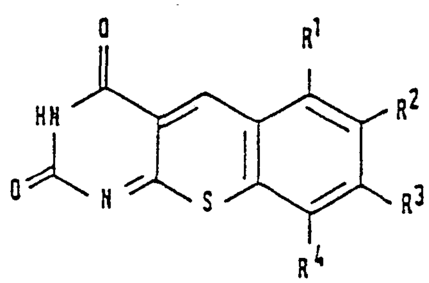

(II)

worin

$R^1$, $R^3$ und $R^4$ gleich oder verschieden sind und einzeln jeweils ein Wasserstoff- oder Halogenatom, eine Carboxylgruppe, eine Alkyl-, Alkoxy- oder Alkoxycarbonylgruppe, worin die Alkylgruppen jeweils 1 bis 6 Kohlenstoffatome enthalten, bedeuten und

$R^2$ eine Carboxyl-, eine Alkoxy- oder Alkoxycarbonylgruppe mit jeweils 1 bis 6 Kohlenstoffatomen in der Alkoxygruppe ist

oder jeweils zwei der Reste

$R^1$, $R^2$, $R^3$ und $R^4$ gemeinsam einen ankondensierten aromatischen Rest bedeuten.

**Claims**

1. A photopolymerizable mixture which contains, as essential components,

a) a polymeric binder,

b) a polymerizable compound having at least two terminal ethylenically unsaturated groups and a boiling point of more than 100 °C, and

c) a polynuclear N-heterocyclic compound as a photoinitiator,

characterized in that the N-heterocyclic compound has the general formula II

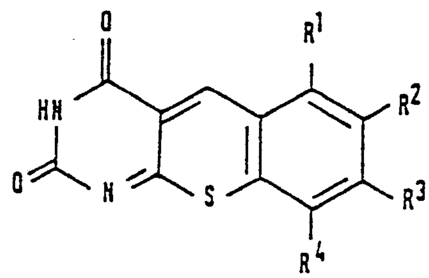

(II)

wherein $R^1$, $R^2$, $R^3$ and $R^4$ are identical or different and individually each denote a hydrogen or halogen atom, a carboxy group, an alkyl, alkoxy or alkoxycarbonyl group in which each alkyl group has from 1 to 6 carbon atoms, or a combination of two of them each denotes a fused aromatic radical.

2. Photopolymerizable mixture as claimed in Claim 1, characterized in that it contains a compound of the formula II wherein one of the radicals $R^1$ to $R^4$ is a hydrogen atom.

3. Photopolymerizable mixture as claimed in Claim 2, characterized in that it contains a compound of the formula II wherein two or three of the radicals $R^1$ to $R^3$ are hydrogen atoms.

4. 1,3-diaza-9-thia-anthracene-2,4-diones of the general formula (II)

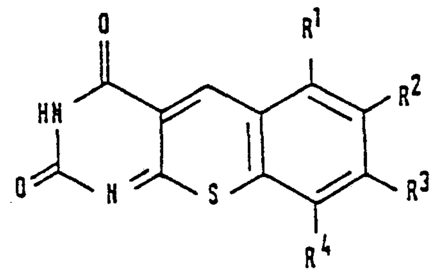

(II)

wherein

$R^1$, $R^3$ and $R^4$ are identical or different and individually each denote a hydrogen or halogen atom, a carboxy group, an alkyl, alkoxy or alkoxycarbonyl group in which each alkyl group has from 1 to 6 carbon atoms, and

$R^2$ denotes a carboxyl, an alkoxy or alkoxy carbonyl group, in which each alkoxy group has from 1 to 6 carbon atoms,

or a combination of two of the radicals

$R^1$, $R^2$, $R^3$ and $R^4$ each denotes a fused aromatic radical.

**Revendications**

1. Mélange photopolymérisable qui contient comme constituants essentiels :

a) un liant polymère,

b) un composé polymérisable avec au moins 2 groupes terminaux à insaturation éthylénique et avec un point d'ébullition supérieur à 100 °C, et

c) comme photoinitiateur, un composé N-hétérocyclique à plusieurs noyaux, caractérisé en ce que le composé N-hétérocyclique correspond à la formule II

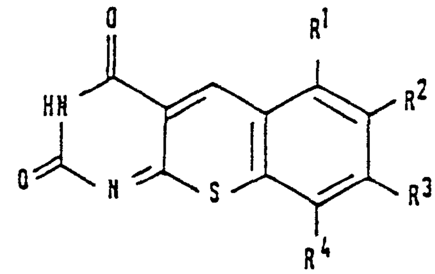

(II)

dans laquelle $R^1$, $R^2$, $R^3$ et $R^4$ sont identiques ou différents et représentent chacun un atome d'hydrogène ou un atome d'halogène, un groupe carboxyle, un groupe alkoyle, alcoxy, ou alcoxycarbonyle dans lesquels les groupes alkoyle ont chacun de 1 à 6 atomes de carbone, ou deux des radicaux $R^1$ à $R^4$ représentent ensemble un radical aromatique condensé.

2. Mélange photopolymérisable selon la revendication 1, caractérisé en ce qu'il contient un composé de formule II, dans lequel l'un des radicaux $R^1$ à $R^4$ est un atome d'hydrogène.

3. Mélange photopolymérisable selon la revendication 2, caractérisé en ce qu'il contient un composé de formule II, dans lequel 2 ou 3 des radicaux $R^1$ à $R^3$ sont des atomes d'hydrogène.

4. 1,3-diaza-9-thia-anthracène-2,4-diones de formule générale II

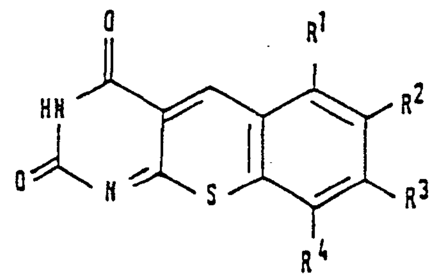

(II)

dans laquelle,

$R^1$, $R^3$ et $R^4$ sont identiques ou différents et représentent chacun un atome d'hydrogène ou d'halogène, un groupe carboxyle, un groupe alkoyle, alcoxy ou alcoxycarbonyle, dans lesquels les groupes alkoyle contiennent chacun de 1 à 6 atomes de carbone, et

$R^2$ est un groupe carboxyle, un groupe alcoxy ou alcoxycarbonyle ayant chacun de 1 à 6 atomes de carbone dans le groupe alcoxy,

ou deux quelconques des radicaux,

$R^1$, $R^2$, $R^3$ et $R^4$ représentent ensemble un radical aromatique condensé.

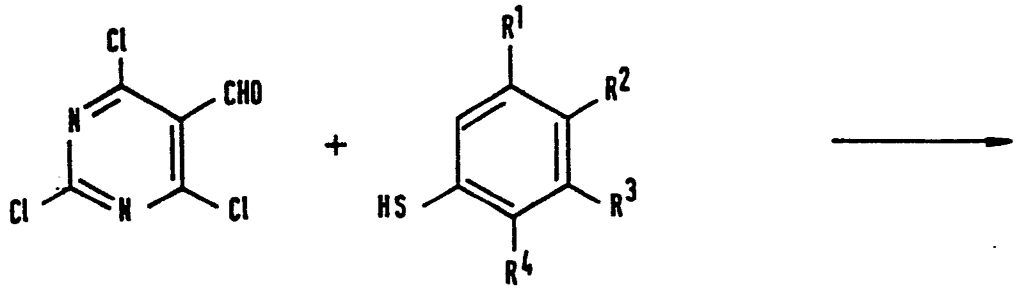

(I)

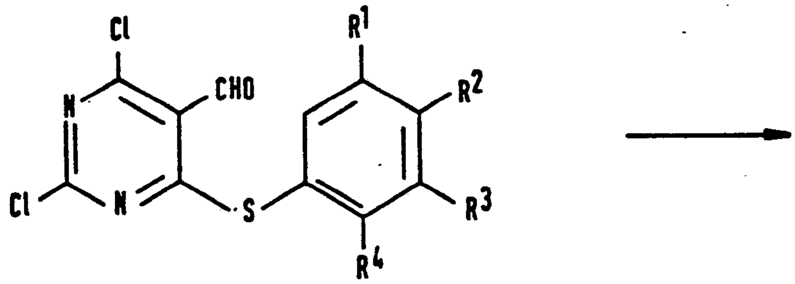

(II)